# EUROPEAN PATENT APPLICATION

(11) **EP 4 297 039 A1**
(43) Date of publication of application: **27.12.2023**
(21) Application number: 22187507.3
(22) Date of filing: 28.07.2022
(51) Int. Cl.: G16H 10/60, G16H 15/00, G16H 50/70, G16H 10/40, G06F 16/332, G06F 16/33, G06N 3/08

(54) **DATA PROCESSING METHOD, DEVICE AND STORAGE MEDIUM**

(30) Priority: 21.06.2022 WO PCT/CN2022/100008
(71) Applicant: Koninklijke Philips N.V., 5656 AG Eindhoven (NL)
(72) Inventor: HAN, Yang, Eindhoven (NL); ZONG, Hui, Eindhoven (NL); ZHANG, Zeyu, 5656AG Eindhoven (NL); LI, Zuo Feng, 5656AG Eindhoven (NL); GUAN, Steven, Eindhoven (NL)
(74) Representative: Philips Intellectual Property & Standards

(57) **Abstract**

Embodiments of the present disclosure relate to a data processing method, device and storage medium. A data processing method includes obtaining a text of a data record. The method further includes obtaining a questionnaire including at least one question, the at least one question being associated with a data type of data in a data record. The method further includes selecting, based on the data type, a data extraction module from a plurality of data extraction modules being collectively configured to extract data of a plurality of data types, the selected data extraction module being configured to extract data of the data type from the text. The method further includes extracting, by the selected data extraction module, data from the text of the data record. Embodiments of the present disclosure provide a general and accurate data processing scheme.

## Description

### FIELD OF THE INVENTION

Embodiments of the present disclosure generally relate to data processing, and more particularly, to a method, a device and a storage medium for data processing.

### BACKGROUND OF THE INVENTION

One important component used to facilitate the use of data is the information extraction (IE) task, which automatically extracts and encodes information from a text of a data record. However, there are various categories of data records, and the existing methods or systems generally deal with a certain category of data records. Currently, there is no method or system that can process multiple categories of data records at the same time for information extraction.

### SUMMARY OF THE INVENTION

According to embodiments of the present disclosure, a method, a device, a system, a computer program product and a storage medium for data processing are provided.

In a first aspect of the present disclosure, a data processing method is provided. The method includes obtaining a text of a data record. The method may further include obtaining a questionnaire including at least one question, the at least one question being associated with a data type of data in a data record. The method may further include selecting, based on the data type, a data extraction module from a plurality of data extraction modules being collectively configured to extract data of a plurality of data types, the selected data extraction module being configured to extract data of the data type from the text. The method may further include extracting, by the selected data extraction module, data from the text of the data record.

In some embodiments, obtaining a questionnaire includes: determining a category of the data record, and selecting, based on the category of the data record, a questionnaire from a plurality of candidate questionnaires as the questionnaire.

In some embodiments, determining the category of the data record includes: obtaining a word library including a plurality of words associated with categories of the data record; searching the text of the data record based on the word library; and in response to determining that a word in the word library is found in the text, determining the category of the data record based on the word.

In some embodiments, selecting a questionnaire from a plurality of candidate questionnaires includes: in response to determining that the category of the data record is a data record including at least one table, selecting a questionnaire including a question associated with a table; and in response to determining that the category of the data record is a data record including at least one paragraph, selecting a questionnaire including a question associated with a paragraph.

In some embodiments, the question in association with the data record including at least one table is associated with one or more of: an item of a medical laboratory examination, an object value for the item, a unit for the object value, a reference value for the item, and an abnormal indication. The question in association with the data record including at least one paragraph is associated with one or more of: a datetime data type, a choice data type, a Boolean data type, a string data type, and a quantity data type.

In some embodiments, the data record including at least one table includes a medical laboratory examination report having the at least one table. The data record including at least one paragraph includes a medical imaging report having the at least one paragraph.

In some embodiments, the at least one question includes at least a first question associated with a first data type and a second question associated with a second data type different from the first data type. The method further comprises: selecting, based on the first data type, a first data extraction module associated with the first data type from the plurality of data extraction modules; extracting, by the first data extraction module, data from the text of the data record; selecting, based on the second data type, a second data extraction module associated with the second data type from the plurality of data extraction modules; and extracting, by the second data extraction module, data from the text of the data record.

In some embodiments, the plurality of data extraction modules comprises at least two of the following: a datetime module associated with a datetime data type, the datetime module being configured to extract data of the datetime data type from the text; a text classifier associated with a choice data type or a Boolean data type, the text classifier being configured to extract data of the choice data type or the Boolean data type from a paragraph of the text; a question and answers module associated with a string data type or a quantity data type, the question and answers module being configured to extract data of the string data type or the quantity data type from the text as an answer to the question; and a named entity recognition module associated with a table, the named entity recognition module being configured to extract an entity corresponding to an entity category from a table area of the text.

In some embodiments, the text classifier is configured to: select a paragraph in the text of the data record; segment the paragraph into multiple sentences; determine a data attribute based on the question; determine a semantical relationship between each of the multiple sentences and the data attribute; and determine an answer to the question based on the semantical relationship.

In some embodiments, the named entity recognition module includes a conditional random field model. The conditional random field model is configured to: detect an entity in the table area; determine that the detected entity matches an entity category of a plurality of entity categories, wherein the plurality of entity categories includes one or more of: an item of a medical laboratory examination, an object value for the item, a unit for the object value, a reference value for the item, and an abnormal indication; and generate a label indicating the detected entity and the matched entity category.

In some embodiments, the question and answers module is configured to: determine a data attribute based on the question; determine a location of one or more characters related to the data attribute in the text; and extract data of the string data type or the quantity data type from the text based on the location.

In some embodiments, the method further includes outputting the questionnaire filled with the extracted data.

In some embodiments, the selected data extraction module is generated by training a machine learning model with a training data set of the data type. The training data set includes standard data obtained from a plurality of data records.

In a second aspect of the present disclosure, an electronic device is provided, which may be adapted to perform the previously described method. The electronic device includes at least one processing unit and a memory. The memory is coupled to the at least one processing unit and stores computer program instructions therein. The instructions, when executed by the at least one processing unit, causes the electronic device to perform the method described according to the first aspect of the present disclosure.

In a third aspect of the present disclosure, there is provided a computer-readable storage medium having program code stored thereon. The program code is configured, upon execution, to cause an apparatus to perform the method described according to the first aspect of the present disclosure.

Embodiments of the present disclosure may combine various data extraction modules into one hybrid module and call the most relevant data extraction module based on a specific data type of data to be extracted. Thus, embodiments of the present disclosure may deal with various categories of data records and various data types. Moreover, because the most relevant data extraction module is used for a corresponding data type, the extracted result is more accurate. In addition, according to embodiments of the present disclosure, the information extraction (IE) task is driven by questions, rather than based on data extracted by a fixed information extraction model (e.g., a named entity recognition model). Thus, embodiments of the present disclosure can efficiently process the text.

The current concepts will be elaborated upon further in Detailed Description section. This Summary is not intended to identify key features or essential features of the claimed subject matter, nor is it intended to be used to limit the scope of the claimed subject matter.

### BRIEF DESCRIPTION OF THE DRAWINGS

Through the following detailed descriptions with reference to the accompanying drawings, the above and other objectives, features and advantages of the example embodiments disclosed herein will become more comprehensible. In the drawings, several example embodiments disclosed herein will be illustrated in an example and in a non-limiting manner, wherein:
Fig. 1 illustrates a schematic diagram of an example environment for data processing according to an embodiment of the present disclosure;
Fig. 2 illustrates an example process for training and evaluating a machine learning model according to an embodiment of the present disclosure;
Fig. 3 illustrates a fine-tuned process of a machine learning model according to an embodiment of the present disclosure;
Fig. 4 illustrates a flowchart of a method of determining an answer to a question associated with a Boolean data type according to an embodiment of the present disclosure;
Fig. 5 illustrates an example process for determining an answer to a question associated with a Boolean data type according to an embodiment of the present disclosure;
Fig. 6 illustrates an example process for determining an answer to a question associated with a string data type or a quantity data type according to an embodiment of the present disclosure;
Fig. 7 illustrates a flowchart of a data processing method according to an embodiment of the present disclosure; and
Fig. 8 illustrates a schematic block diagram of an example device that may be used to implement embodiments of the present disclosure.

Throughout the drawings, the same or similar reference symbols are used to indicate the same or similar elements.

### DETAILED DESCRIPTION OF EMBODIMENTS

The individual features of the various embodiments, examples, and implementations disclosed within this document can be combined in any desired manner that makes technological sense. Furthermore, the individual features are hereby combined in this manner to form all possible combinations, permutations and variants except to the extent that such combinations, permutations and/or variants have been explicitly excluded or are impractical. Support for such combinations, permutations and variants is considered to exist within this document.

It should be understood that the specialized circuitry that performs one or more of the various operations disclosed herein may be formed by one or more processors operating in accordance with specialized instructions persistently stored in memory. Such components may be arranged in a variety of ways such as tightly coupled with each other (e.g., where the components electronically communicate over a computer bus), distributed among different locations (e.g., where the components electronically communicate over a computer network), combinations thereof, and so on.

Some preferable embodiments will be described in more detail with reference to the accompanying drawings, in which the preferable embodiments of the present disclosure have been illustrated. However, the present disclosure can be implemented in various manners, and thus should not be construed to be limited to the embodiments disclosed herein. Rather, those embodiments are provided for thorough and complete understanding of the present disclosure, and completely conveying the scope of the present disclosure to those skilled in the art.

The term "comprise" and its variations used in the present disclosure mean including in an open-ended sense, i.e. "include without limitation". Unless otherwise specified, the term "or" means "and/or". The term "based on" means "at least partially based on". The terms "one exemplary embodiment" and "one embodiment" represent "at least one embodiment"; the term "another embodiment" represents "at least one another embodiment". The terms "first", "second" and the like may refer to different or the same objects. Other explicit and implicit definitions might further be included in the following description.

Embodiments of the present disclosure are usually described based on a medical record, but principles of the present disclosure are also applicable to other categories of data records.

To facilitate the use of data, it is desired to automatically extract information from a text of a data record. However, there are various categories of data records, and a category of data record may contain data of a different data type from another category of data record. Moreover, the same data record may contain data of a variety of data types. The existing methods or systems generally deal with a certain category of data records or a certain data type of data. Currently, there is no method or system that can process multiple categories of data records or multiple data types of data at the same time for information extraction.

For example, a medical record is an important category of the data record. In developed country, with the rapid adoption of digitalization, there is always a desire to harvest information and knowledge from digital health record or reports to support automated systems at the end to enable clinical research activities. One critical component used to facilitate the use of patient data is the information extraction (IE) task, which automatically extracts and encodes clinical information from a text of a medical record.

However, there are various categories of reports in the hospital. A medical record may include a variety of types of "notes" entered overtime by healthcare professionals, recording observations and administration of drugs and therapies, radiology test results, laboratory examination results, etc.

Typically, a fixed information extraction model is used to process all data types of data. In the field of clinical information extraction, most methods are focused on using named entity recognition (NER) task as their first step. All other downstream tasks are based on the NER task extracted results. However, the NER task is not suitable for all data types, which results in inaccurate extraction results for some data types.

In order to perform information extraction over various categories of data records (e.g., medical records), there is a need to build a hybrid module which will call the most relevant module to process a specific data type of data based on the task at hand.

Embodiments of the present disclosure provide a data processing method, device and system that integrate different types of data extraction modules to serve different data types of data. Embodiments of the present disclosure can select a more suitable data extraction module for a specific data type of data.

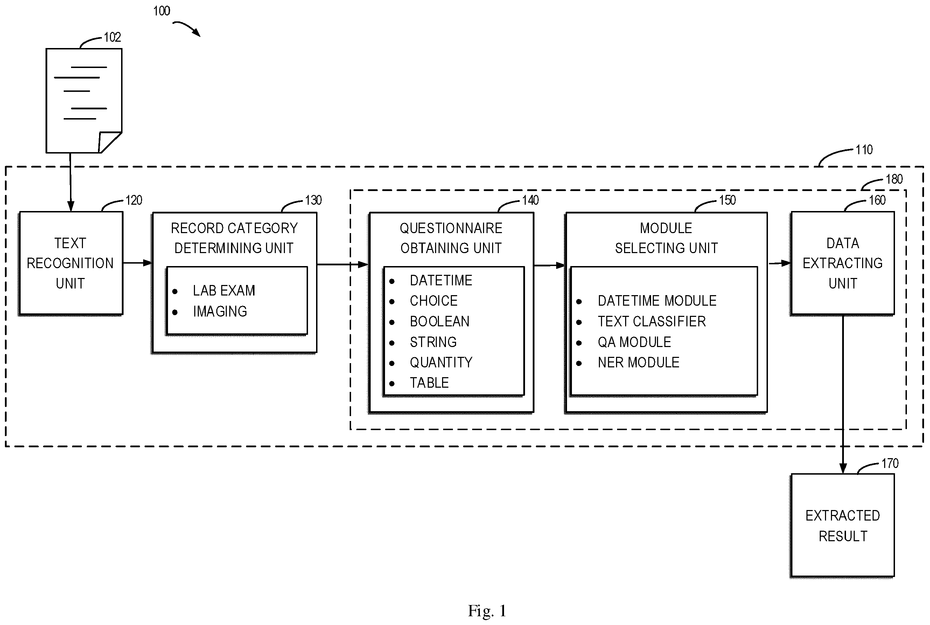

Fig. 1 illustrates a schematic diagram of an example environment 100 for data processing according to an embodiment of the present disclosure. It would be appreciated that the structure and function of the example environment 100 are depicted only for the purpose of illustration without suggesting any limitation to the scope of the present disclosure. Embodiments of the present disclosure may be embodied in different structures and/or functions.

As shown in Fig. 1, the example environment 100 may include a data processing system 110. The data processing system 110 may receive a data record 102 and output an extracted result 170 including data extracted from the data record 102. The extracted result 170 may also include encoded data generated based on the data extracted from the data record 102. The data record 102 may be a data record of any type. For example, the data record 102 may be a written (paper note), physical (image film) or digital record. If the data record 102 is a written or physical record, it can be scanned to obtain a digital version.

The data processing system 110 may include a text recognition unit 120 that can recognize a text from a digital version of the data record 102. For example, the text recognition unit 120 may perform an optical character recognition (OCR) on the data record 102 to obtain a text of the data record 102. The data processing system 110 may extract information from the text of the data record 102. The text recognition unit 120 is optional and the data processing system 110 may directly receive a text of the data record 102.

The data processing system 110 may include a multi-type Natural Language Processing (NLP) module 180. The multi-type NLP module 180 integrates various (off-the shelf) data extraction modules into one hybrid module and will call the most relevant data extraction module based on a specific data type of data to be processed. In this way, the data processing system 110 can process multiple categories of data records or multiple data types of data for information extraction. A data extraction module of the hybrid module is specifically built for a specific data type, such that an accurate extraction result can be obtained.

The multi-type NLP module 180 may include a questionnaire obtaining unit 140. When receiving the text of the data record 102, the questionnaire obtaining unit 140 may determine a questionnaire for the data record 102. The questionnaire may be predefined or temporarily generated. For example, in response to receiving the text of the data record 102, the questionnaire obtaining unit 140 may display a blank questionnaire on a graphical user interface. A user may input one or more interested question into the blank questionnaire, to generate the questionnaire. In such embodiments, the information extraction (IE) task is driven by questions, rather than based on data extracted by a fixed information extraction model (e.g., a named entity recognition model). For a question in the questionnaire, the data processing system 110 extracts data from the text. As such, the data processing is more targeted and more efficient.

The questionnaire may include at least one question associated with a data type of data in a data record. The questionnaire may include multiple questions associated with multiple data types. Questions in the questionnaire may be associated with various data types such as date, time, datetime, choice, Boolean, string, quantity and table. For a data type, there may be at least one question in the questionnaire. As described below, a corresponding data extraction module may be loaded for each data type.

The multi-type NLP module 180 may include a module selecting unit 150. For a question in the questionnaire, the module selecting unit 150 may select a data extraction module from a plurality of data extraction modules based on the data type associated with the question. The plurality of data extraction modules include different types of data extraction modules and thus are collectively configured to extract data of a plurality of data types. For example, the different types of data extraction modules in the hybrid module may include: a datetime module associated with a datetime data type, a text classifier associated with a choice data type or a Boolean data type, a question and answers (QA) module associated with a string data type or a quantity data type, and a named entity recognition (NER) module associated with a table. The selected data extraction module is configured to extract data of the data type associated with the question from the text of the data record 102.

The multi-type NLP module 180 may include a data extracting unit 160. After the module selecting unit 150 determines the selected data extraction module for the question, the data extracting unit 160 may use the selected data extraction module to extract data from the text of the data record 102, to obtain an answer to the question. The data extracting unit 160 may generate the extracted result 170. The extracted result 170 may include the questionnaire filled with the extracted data.

Because the multi-type NLP module 180 is a hybrid module including different types of data extraction modules, the data processing system 110 is able to serve extraction of data of different data types. As a result, the data processing system 110 can deal with various data types and various categories of data records, and thus is more general. A most suitable data extraction module is loaded for a specific data type, which ensures a more accurate information extraction result.

In some optional embodiments, the questionnaire obtaining unit 140 provides a questionnaire including a first question associated with a first datatype and a second question associated with a second data type different from the first data type. For the first question and based on the first data type, the module selecting unit 150 selects a first data extraction module associated with the first data type. For the second question and based on the second data type, the module selecting unit 150 selects a second data extraction module associated with the second data type. The data extracting unit 160 uses the first data extraction module to extract data of the first data type from the text, giving an answer to the first question. The data extracting unit 160 also uses the second data extraction module to extract data of the second data type from the text, giving an answer to the second question.

Optionally, the data processing system 110 may include a record category determining unit 130. The record category determining unit 130 may determine a category of the data record 102. The record category determining unit 130 may determine whether the data record belongs to a category associated with a medical examination approach (e.g., a laboratory examination or imaging examination) or an examined body part. For example, the record category determining unit 130 may determine whether the data record is a medical laboratory examination (LAB EXAM) report or a medical imaging report. The medical imaging report may include a radiology report, an ultrasonic report, etc. For example, the record category determining unit 130 may also determine whether the data record is a medical imaging report on e.g. thyroid, breast, or heart.

If the category of the data record is determined, the questionnaire obtaining unit 140 may determine a questionnaire for the data record based on the category, such that the questions in the questionnaire are more relevant to the data record. Such embodiments further provide a more targeted processing, and more useful information can be extracted.

In some optional embodiments, the questionnaire obtaining unit 140 may select a questionnaire from a plurality of candidate questionnaires as the questionnaire, based on the category of the data record. In this way, the questionnaire may better matches with the data record, and more useful information can be obtained from the text of the data record 102.

In some optional embodiments, to determine the category of the data record 102, the record category determining unit 130 may use a word library. The word library may be predefined. The word library includes a plurality of word sets associated with different categories of the data record. For example, a first word set of the plurality of word sets includes at least one word or phrase associated with a first record category, and a second word set of the plurality of word sets includes at least one word or phrase associated with a second record category. The record category determining unit 130 may search the text of the data record 102 based on the word library. According to which word in the word library being found in the text, the record category determining unit 130 may determine the record category based on the found word.

Specifically, in the word library, the word "imaging" is associated with a medical imaging report, and the word "blood routine" is associated with a medical laboratory examination (LAB EXAM) report. If the word "imaging" is found in the text, the record category determining unit 130 may determine that the data record 102 is a medical imaging report. If the word "blood routine" is found in the text, the record category determining unit 130 may determine that the data record 102 is a medical laboratory examination (LAB EXAM) report. Further, the record category determining unit 130 may determine the record category based on a location of the found word and word frequency. If the word "imaging" is found in the head section of the data record 102, the data record 102 may be determined as a medical imaging report. If the word "heart" occurs many times, the data record 102 may be determined to be related to the heart of a patient. Such embodiments are based on rules, and are simple and feasible. Alternatively, the record category determining unit 130 may use a machine learning model to determine the category of the data record 102.

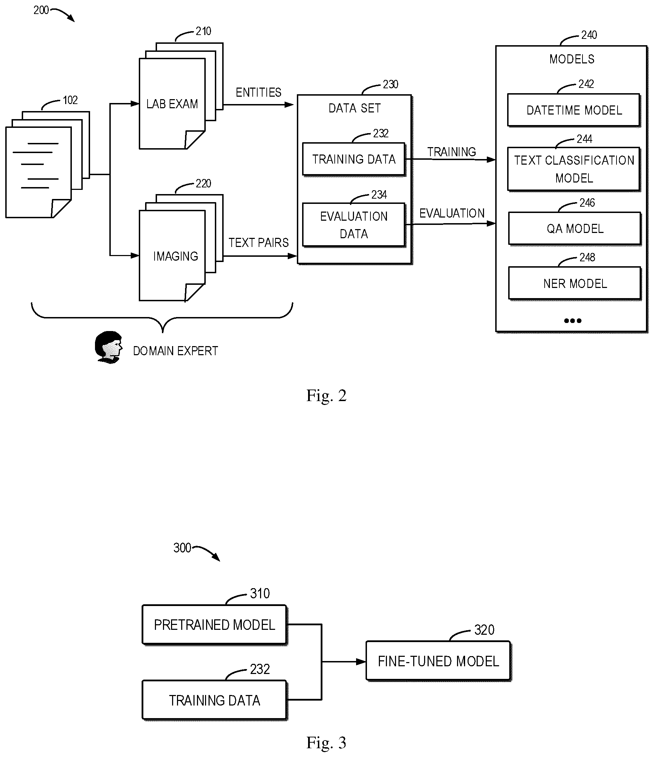

Fig. 2 illustrates an example process 200 for training and evaluating a machine learning model according to an embodiment of the present disclosure. The plurality of data extraction modules in the multi-type NLP module 180 may include machine learning models 240. In detail, the datetime module may include a datetime model 242, the text classifier may include a text classification model 244, the question and answers (QA) module may include a QA model 246, and the named entity recognition (NER) module may include a NER model 248. Fig. 2 illustrates an example process 200 for training and evaluating such machine learning models to obtain the trained (off-the shelf) datetime model 242, text classification model 244, QA model 246, and NER model 248. Alternatively, data extraction modules may be completely rule-based modules without a machine learning model, especially such as the datetime module.

As shown in Fig. 2, training and evaluation of the machine learning models 240 are based on a plurality of data records 102. The plurality of data records 102 may include different languages such that the plurality of data extraction modules can be applied to different languages. The plurality of data records 102 may include only Chinese, such that the plurality of data extraction modules are dedicated to data records in Chinese. Alternatively, the plurality of data records 102 may include only English, such that the plurality of data extraction modules are dedicated to data records in English. The machine learning models 240 can also be trained using other languages. The scope of the present disclosure is not limited in language.

The plurality of data records 102 are labeled by a domain expert (e.g., a doctor) or a trained machine learning model, so as to identify categories of the plurality of data records 102. For example, a data record 102 may be identified to be a medical laboratory examination (LAB EXAM) report 210 or a medical imaging report 220. A medical LAB EXAM report 210 typically includes at least one table that has a form of an array. The table typically includes multiple entities. The entities in the medical LAB EXAM report 210 and the corresponding entity categories are labeled by the domain expert or the trained machine learning model. A medical imaging report 220 typically includes at least one one paragraph. Text pairs are labeled by the domain expert or the trained machine learning model from the paragraph of the medical imaging report 220. The text of the plurality of data records 102 and the labeled record categories, entities, corresponding entity categories, and text pairs are added into the data set 230. Thus, the data set 230 includes standard data (also called as ground truth) for training or evaluating the machine learning models 240.

The data set 230 may be divided into training data 232 and evaluation data 234. The training data 232 is used for training a model of the machine learning models 240. The evaluation data 234 is used for for evaluating the accuracy of a model of the machine learning models 240 for quality assurance purposes. The training data 232 and the evaluation data 234 may be classified according to data types of data, and a model of the machine learning models 240 may be trained or evaluated with data of the corresponding data type. In this way, the trained model can be particularly suitable for the corresponding data type. This improves the accuracy of the extracted result 170.

Fig. 3 illustrates a fine-tuned process 300 of a machine learning model according to an embodiment of the present disclosure. A pre-trained machine learning model 310 may be available. In the fine-tuned process 300, the training data 232 described with respect to Fig. 2 may be used to fine tune the pre-trained machine learning model 310, to obtain a fine-tuned model 320. For example, the text classification model 244 may be developed based on a Bert model. A pre-trained Bert model may be fine-tuned with the training data 232, to obtain a fine-tuned Bert model as the text classification model 244. Various data extraction modules included in the multi-type NLP module 180 as shown in Fig. 1 will be described below. The datetime module is configured to extract data of the datetime data type from the text of the data record. Herein, the term "datetime" may refer to date and/or time. A question in association with the datetime data type is mainly associated with one of: examination datatime, sampling datatime, reception datatime, report datatime, review datatime, and printing datatime, etc. The datetime module is configured to convert the string representation of a date and time value to its datetime equivalent having a specific format. For example, the string representation of "June 6, 2019" may be converted by the datetime module to "2019-6-6". The datetime module may be implemented by a datetime parser. The datetime parser may be developed with Python.

An electronic medical record contains multiple sections: the head section containing basic information like name, gender, age, etc.; the middle section containing clinical findings, measurements and diagnostic; and the tail section generally containing datetime information and doctor information. Most of the information is dived in the middle section. In the case of a medical imaging report, the middle section is organized as at least one paragraph having multiple sentences. In the case of a medical laboratory examination report, the middle section is organized as at least one table having multiple columns. Each column corresponds to an entity category such as an item of a medical laboratory examination, an object value for the item, a unit for the object value, a reference value for the item, or an abnormal indication.

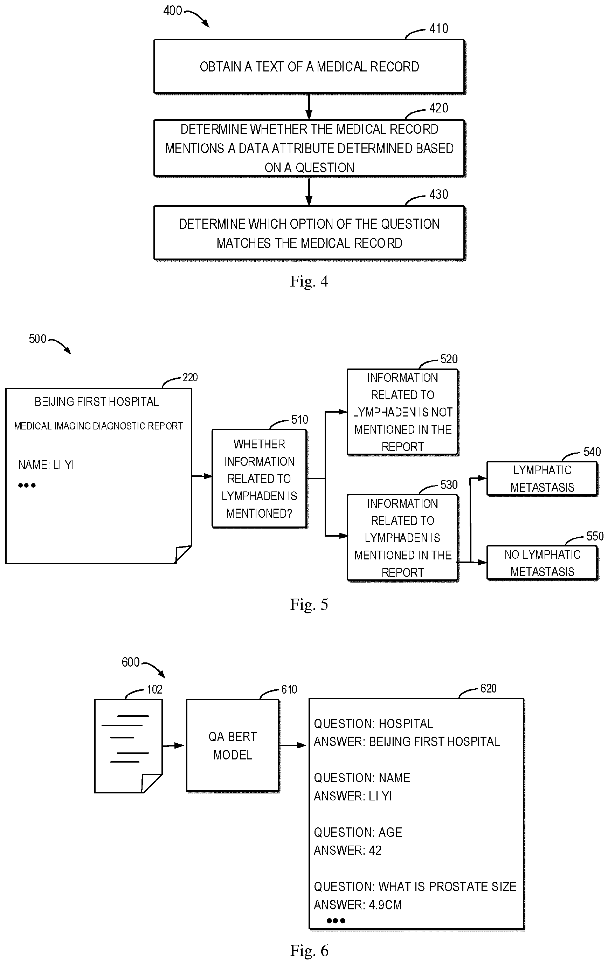

The text classifier is configured to extract data of the choice data type or the Boolean data type from a paragraph of the text of the data record. Data of the choice data type or the Boolean data type is usually contained in the at least one paragraph of a data record. If it is determined that the category of the data record is a data record including at least one paragraph, a questionnaire including a question associated with a paragraph may be selected and loaded. The question may be associated with one or more of: a datetime data type, a choice data type, a Boolean data type, a string data type, and a quantity data type. If the question is associated with a choice data type or a Boolean data type, the text classifier may be selected. It is noted that the at least one paragraph of a data record may include data of other data types such as a datetime data type. As an example, the at least one paragraph of a data record may include a sentence "compared with the examination result on December 6, 2018, there is no significant change" that includes data of the datetime data type. The application of the text classifier will be described with reference to Figs. 4 and 5.

Fig. 4 illustrates a flowchart of a method 400 of determining an answer to a question associated with a Boolean data type according to an embodiment of the present disclosure. It is to be appreciated that the method 400 may further include additional blocks not shown and/or omit some blocks as shown. The scope of the present disclosure is not limited in this regard. Fig. 5 illustrates an example process 500 for determining an answer to a question associated with a Boolean data type according to an embodiment of the present disclosure. For example, the method 400 and the example process 500 may be implemented by the data extracting unit 160 as shown in Fig. 1 using a text classifier. The example process 500 is an example implementation of the method 400, and thus Figs. 4 and 5 will be described together.

At 410 of Fig. 4, the text classifier obtains a text of a medical record. As described above, the text of a medical record may be generated via an optical character recognition (OCR). Fig. 5 shows a medical imaging report 220 as an example of the medical record.

At 420 of Fig. 4, the text classifier determines whether the medical record mentions a data attribute. The data attribute may be determined based on the question. For example, if the question associated with a Boolean data type is "whether there is lymphatic metastasis", the data attribute may be determined as "lymphaden". Alternatively, the data attribute may also be determined as "lymphatic metastasis." Alternatively, the question itself may be determined as the data attribute. As shown in 510 of Fig. 5, the text classifier determines whether information related to lymphaden is mentioned in the medical imaging report 220.

The text classifier can be used to extract a most relevant sentence from the at least one paragraph of the medical imaging report 220, and also can give an answer to a question associated with a Boolean data type or a choice data type. The text classifier may include two functions: sentence segmentation and sentence classification.

In some optional embodiments, the text classifier may select a paragraph in the text of the medical imaging report 220. The text classifier may segment the paragraph into multiple sentences with a developed sentence segmentation function. The text classifier may also determine a semantical relationship between each of the multiple sentences and the data attribute. Specifically, the text classifier may combine each sentence and the data attribute into a text pair. The text classifier may include a text pair language model (e.g., BERT model. The BERT model is sometimes spelled as Bert in this application). The text pair is used as an input of the text pair language model. An output of the text pair language model indicates the semantical relationship between the sentence and the data attribute in the text pair. The output of the text pair language model may be 0, -1, or 1. The output "0" means that the input text pair is not semantically related, that is, the sentence is not semantically related to the data attribute. In other words, there is no answer for the data attribute (or the question) in this sentence. The output "-1" means opposite semantics, that is, the sentence is opposite semantics to the data attribute. The output "1" means same semantics, that is, the sentence is the same semantics as the data attribute. If the medical imaging report 220 includes more than one paragraph, the above process is performed for each paragraph. In this way, the most relevant sentence for the data attribute can be located, to facilitate determining an answer to the question.

Return to Fig. 4, at 430, the text classifier may determine which option of the question matches the medical record. As shown in Fig. 5, for the question associated with a Boolean data type, there are two options: lymphatic metastasis at 540 and no lymphatic metastasis at 550. The text classifier may determine an answer to the question based on the semantical relationship indicated by the output of the text pair language model. If the text pair language model outputs "0" for all sentences in the at least paragraph, at 520 of Fig. 5, the text classifier may determine that information related to lymphaden is not mentioned in the medical imaging report 220. Otherwise, if the output for one or more sentence is "1" or "-1", at 530 of Fig. 5, the text classifier may determine that information related to lymphaden is mentioned in the medical imaging report 220. In this way, the text classifier can search for the relevant sentences from the at least one paragraph, so as to give an answer to the question. If the text pair language model outputs "1", at 540 of Fig. 5, the text classifier may determine that the patient has lymphatic metastasis. If the text pair language model outputs "-1", at 550 of Fig. 5, the text classifier may determine that the patient has no lymphatic metastasis.

It is possible that more than one data attribute may be determined based on one question. When the question is associated with the choice data type and multiple options are provided for the question in the questionnaire, each option may be determined as a data attribute. For example, if the question is "whether the thyroid nodule is benign, malignant, or undifferentiated" and three options "benign," "malignant" and "undifferentiated" are provided in the questionnaire, "thyroid" may determined as a data attribute and the three options may determined as data attributes, respectively. The determination of the data attribute may be performed based on rules or performed with a trained machine learning model. Each data attribute of the more than one data attribute determined based on one question may be combined with the sentences in the at least one paragraph to form text pairs. The text classifier may process all these text pairs, to obtain a final answer to the question. That is, the text classifier may determine which option of the three options matches the data record.

The named entity recognition (NER) module is configured to extract an entity corresponding to an entity category from a table area of the text. A table in a data record may include multiple columns. Each column corresponds to an entity category. The table includes at least one entity corresponding to each entity category. If it is determined that the category of the data record is a data record including at least one table, a questionnaire including a question associated with a table may be selected and loaded. For example, the question may be associated with one or more of: an item of a medical laboratory examination, an object value for the item, a unit for the object value, a reference value for the item, and an abnormal indication. Accordingly, the NER module may be selected.

The NER module is configured to have the entity categories such as an item of a medical laboratory examination, an object value for the item, a unit for the object value, a reference value for the item, and an abnormal indication. In this way, the NER module is particularly suitable for information extraction for the medical laboratory examination (LAB EXAM) report 210. The NER module may be configured to have other entity categories, to be able to deal with other record categories including a table. This further improves the versatility of the data processing system 110.

In some optional embodiments, the NER module may include a conditional random field (CRF) model. The CRF model employs a statistical modeling method, and is often applied in pattern recognition and machine learning and used for structured prediction.

In some optional embodiments, the CRF model may select a table area in the text of the data record, detect an entity in the table area, and determine whether the detected entity matches an entity category of a plurality of entity categories. The plurality of entity categories includes one or more of: an item of a medical laboratory examination, an object value for the item, a unit for the object value, a reference value for the item, and an abnormal indication. In this way, the CRF model is particularly suitable for information extraction for the medical laboratory examination (LAB EXAM) report 210. The CRF model may also generate a label indicating the detected entity and the matched entity category.

The CRF model may perform sequence labelling on the text of the data record 102, especially on a table area of the text. The input sequence of the CRF model includes characters of the table area. The output sequence of the CRF model includes a plurality of labels at locations corresponding to the characters. The plurality of labels indicates the detected entity and the matched entity category. In this way, the CRF model can obtain data such as an item of a medical laboratory examination, an object value for the item, a unit for the object value, a reference value for the item, and an abnormal indication. The CRF model can label the whole table area or even the whole text at once, without segmentation for the table area and the text.

For example, the question may be "an item of a medical laboratory examination." The CRF model may recognize the entities belonging to the entity category "an item of a medical laboratory examination" as answers to the question. For a blood routine report, the answers to the question may include white blood cell, red blood cell, hemoglobin, etc.

The question and answers (QA) module is built specifically to extract data of the string data type or the quantity data type from the text as an answer to the question. In some optional embodiments, the QA module may determine a data attribute based on the question. The QA module may determine a location of one or more characters related to the data attribute in the text. The QA module may also extract data of the string data type or the quantity data type from the text based on the location.

This QA module may be used to get answer for string and quantity data types with a QA Bert model. The QA Bert model is applying the bidirectional training of Transformer to language modelling. The record text and the data attribute may be combined into a text pair as an input to the QA Bert model. Taking the record text as the context and the data attribute as the question, the QA Bert model will find the most likely text span as answer. For example, the output of the QA Bert model may indicate that the text span from the first character to the third character is related to the data attribute. Then, an answer may be determined based on the text span. The QA Bert model or the QA module can process the whole text at once, without segmentation for the text.

Fig. 6 illustrates an example process 600 for determining an answer to a question associated with a string data type or a quantity data type according to an embodiment of the present disclosure. For example, the example process 600 may be implemented by the data extracting unit 160 as shown in Fig. 1 using a QA module. In the example process 600, the QA module is illustrated as a QA Bert model 610. The data record 102 may be provided to the QA Bert model 610. Based on the output of the QA Bert model 610, the questionnaire 620 filled with the extracted data can be obtained and output. As shown in the questionnaire 620, the QA Bert model 610 can be used to extract data of the string data type, such as "BEIJING FIRST HOSPITAL" and "LI YI", and can also be used to extract data of the quantity data type, such as "42" and "4.9CM".

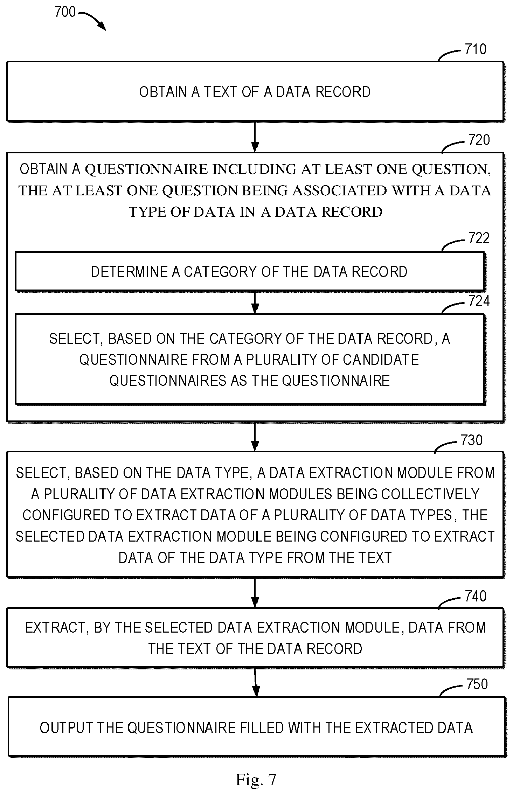

Fig. 7 illustrates a flowchart of a data processing method 700 according to an embodiment of the present disclosure. For example, the method 700 may be implemented by the data processing system 110 as shown in Fig. 1. It is to be appreciated that the method 700 may further include additional blocks not shown and/or omit some blocks as shown. The scope of the present disclosure is not limited in this regard.

At 710, the data processing system 110 may obtain a text of a data record.

At 720, the data processing system 110 may obtain a questionnaire including at least one question. The at least one question is associated with a data type of data in a data record.

In some embodiments, at 722, the data processing system 110 may determine a category of the data record. At 724, based on the category of the data record, the data processing system 110 may select a questionnaire from a plurality of candidate questionnaires as the questionnaire.

In some embodiments, the data processing system 110 may obtain a word library including a plurality of words associated with categories of the data record; search the text of the data record based on the word library; and in response to determining that a word in the word library is found in the text, determine the category of the data record based on the word.

In some embodiments, in response to determining that the category of the data record is a data record including at least one table, the data processing system 110 may select a questionnaire including a question associated with a table. In response to determining that the category of the data record is a data record including at least one paragraph, the data processing system 110 may select a questionnaire including a question associated with a paragraph.

In some embodiments, the question in association with the data record including at least one table is associated with one or more of: an item of a medical laboratory examination, an object value for the item, a unit for the object value, a reference value for the item, and an abnormal indication. The question in association with the data record including at least one paragraph is associated with one or more of: a datetime data type, a choice data type, a Boolean data type, a string data type, and a quantity data type.

In some embodiments, the data record including at least one table includes a medical laboratory examination report having the at least one table, and the data record including at least one paragraph includes a medical imaging report having the at least one paragraph.

At 730, the data processing system 110 may select, based on the data type, a data extraction module from a plurality of data extraction modules. The plurality of data extraction modules is collectively configured to extract data of a plurality of data types. The selected data extraction module is configured to extract data of the data type from the text.

At 740, the data processing system 110 may extract, by the selected data extraction module, data from the text of the data record.

In some embodiments, the at least one question includes at least a first question associated with a first data type and a second question associated with a second data type different from the first data type. Based on the first data type, the data processing system 110 may select a first data extraction module associated with the first data type from the plurality of data extraction modules, and extract, by the first data extraction module, data from the text of the data record. Based on the second data type, the data processing system 110 may select a second data extraction module associated with the second data type from the plurality of data extraction modules, and extract, by the second data extraction module, data from the text of the data record.

In some embodiments, the plurality of data extraction modules comprises at least two of the following: a datetime module associated with a datetime data type, the datetime module being configured to extract data of the datetime data type from the text; a text classifier associated with a choice data type or a Boolean data type, the text classifier being configured to extract data of the choice data type or the Boolean data type from a paragraph of the text; a question and answers module associated with a string data type or a quantity data type, the question and answers module being configured to extract data of the string data type or the quantity data type from the text as an answer to the question; and a named entity recognition module associated with a table, the named entity recognition module being configured to extract an entity corresponding to an entity category from a table area of the text.

In some embodiments, the text classifier is configured to: select a paragraph in the text of the data record; segment the paragraph into multiple sentences; determine a data attribute based on the question; determine a semantical relationship between each of the multiple sentences and the data attribute; and determine an answer to the question based on the semantical relationship.

In some embodiments, the named entity recognition module includes a conditional random field, CRF, model. The conditional random field model is configured to: detect an entity in the table area; determine that the detected entity matches an entity category of a plurality of entity categories, wherein the plurality of entity categories includes one or more of: an item of a medical laboratory examination, an object value for the item, a unit for the object value, a reference value for the item, and an abnormal indication; and generate a label indicating the detected entity and the matched entity category.

In some optional embodiments, the question and answers module is configured to: determine a data attribute based on the question; determine a location of one or more characters related to the data attribute in the text; and extract data of the string data type or the quantity data type from the text based on the location.

At optional step 750, the data processing system 110 may output the questionnaire filled with the extracted data.

In some embodiments, the selected data extraction module is generated by training a machine learning model with a training data set of the data type. The training data set includes standard data obtained from a plurality of data records.

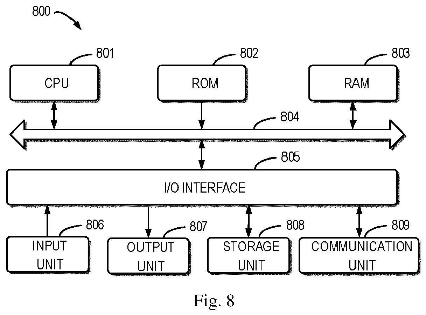

Fig. 8 illustrates a schematic block diagram of an example device 800 that may be used to implement embodiments of the present disclosure. The data processing system 110 as shown in Fig. 1 may be implemented by the example device 800, specifically, by a processing unit 801 of the example device 800.

As indicated, the device 800 includes a central processing unit (CPU) 801 which can perform various appropriate actions and processes based on computer program instructions stored in a read-only memory (ROM) 802 or computer program instructions loaded from a storage unit 808 into a random access memory (RAM) 803. In RAM 803, there are also stored various programs and data required by the device 800 when operating. The CPU 801, ROM 802 and RAM 803 are connected to one another via a bus 804. An input/output (I/O) interface 805 is also connected to the bus 804.

A plurality of components of the device 800 are connected to the I/O interface 805, including: an input unit 806 including a keyboard, a mouse, and the like; an output unit 807, such as various types of displays, loudspeakers, and the like; a storage unit 808 including a magnetic disk, an optical disk, and the like; and a communication unit 809 including a LAN card, a modem, a wireless communication transceiver and so on. The communication unit 809 allows the device 800 to exchange information/data with other devices via a computer network, such as the Internet, and/or various telecommunications networks.

The above-described procedures and processes, such as the methods/processes 200, 300, 400, 500, 600 and 700, can be implemented by the processing unit 801. For example, in some embodiments, the methods/processes 200, 300, 400, 500, 600 and 700 can be implemented as a computer software program which is tangibly embodied on a machine readable medium, for instance, the storage unit 808. In some embodiments, part or all of the computer program can be loaded to and/or installed on the device 800 via the ROM 802 and/or the communication unit 809. The computer program, when loaded to the RAM 803 and executed by the CPU 801, may execute one or more acts of the methods/processes 200, 300, 400, 500, 600 and 700 as described above. Alternatively, the CPU 801 can also be configured to implement the methods/processes 200, 300, 400, 500, 600 and 700 as described above in any other proper manner (for example, by means of firmware).

To sum up, embodiments of the present disclosure provide an improved information extraction scheme. The data processing system 110 includes a plurality of data extraction modules to be selected. The plurality of data extraction modules is collectively configured to extract data of a plurality of data types from a text. Based on the data type of data to be extracted, a most suitable module may be selected, to ensure an accurate extracted result. In case of data of a different data type to be extracted, a different module may be selected. As such, the data processing system 110 and the data processing method 700 are able to deal with various data types and various categories of data records. Hence, embodiments of the present disclosure provide a more general data processing system and method. Because that the data processing task is driven by questions, the data processing is more targeted and more efficient.

The present disclosure may be a method, an apparatus, a system, and/or a computer program product. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may include copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Java, Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to embodiments of the present disclosure. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means (or specialized circuitry) for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein includes an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the figures illustrate the architecture, functionality, and operation of possible implementations of systems, methods and computer program products according to various embodiments of the present disclosure. In this regard, each block in the flowchart or block diagrams may represent a module, segment, or portion of code, which includes one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

The descriptions of the various embodiments of the present disclosure have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A data processing method, comprising:
obtaining a text of a data record;
obtaining a questionnaire including at least one question, the at least one question being associated with a data type of data in a data record;
selecting, based on the data type, a data extraction module from a plurality of data extraction modules being collectively configured to extract data of a plurality of data types, the selected data extraction module being configured to extract data of the data type from the text; and
extracting, by the selected data extraction module, data from the text of the data record.

2. The method of claim 1, wherein obtaining a questionnaire includes:
determining a category of the data record, and
selecting, based on the category of the data record, a questionnaire from a plurality of candidate questionnaires.

3. The method of claim 2, wherein determining the category of the data record includes:
obtaining a word library including a plurality of words associated with categories of the data record;
searching the text of the data record based on the word library; and
in response to determining that a word in the word library is found in the text, determining the category of the data record based on the word.

4. The method of claim 2, wherein selecting a questionnaire from a plurality of candidate questionnaires includes:
in response to determining that the category of the data record is a data record including at least one table, selecting a questionnaire including a question associated with a table; and
in response to determining that the category of the data record is a data record including at least one paragraph, selecting a questionnaire including a question associated with a paragraph.

5. The method of claim 4, wherein
the question in association with the data record including at least one table is associated with one or more of: an item of a medical laboratory examination, an object value for the item, a unit for the object value, a reference value for the item, and an abnormal indication; and
the question in association with the data record including at least one paragraph is associated with one or more of: a datetime data type, a choice data type, a Boolean data type, a string data type, and a quantity data type.

6. The method of claim 4, wherein
the data record including at least one table includes a medical laboratory examination report having the at least one table; and
the data record including at least one paragraph includes a medical imaging report having the at least one paragraph.

7. The method of claim 1, wherein the at least one question includes at least a first question associated with a first data type and a second question associated with a second data type different from the first data type, and the method further comprises:
selecting, based on the first data type, a first data extraction module associated with the first data type from the plurality of data extraction modules;
extracting, by the first data extraction module, data from the text of the data record;
selecting, based on the second data type, a second data extraction module associated with the second data type from the plurality of data extraction modules; and
extracting, by the second data extraction module, data from the text of the data record.

8. The method of claim 1, wherein the plurality of data extraction modules comprises at least two of the following:
a datetime module associated with a datetime data type, the datetime module being configured to extract data of the datetime data type from the text;
a text classifier associated with a choice data type or a Boolean data type, the text classifier being configured to extract data of the choice data type or the Boolean data type from a paragraph of the text;
a question and answers module associated with a string data type or a quantity data type, the question and answers module being configured to extract data of the string data type or the quantity data type from the text as an answer to the question; and
a named entity recognition module associated with a table, the named entity recognition module being configured to extract an entity corresponding to an entity category from a table area of the text.

9. The method of claim 8, wherein the text classifier is configured to select a paragraph in the text of the data record;
segment the paragraph into multiple sentences;
determine a data attribute based on the question;
determine a semantical relationship between each of the multiple sentences and the data attribute; and
determine an answer to the question based on the semantical relationship.

10. The method of claim 8, wherein the named entity recognition module includes a conditional random field model, and the conditional random field model is configured to
detect an entity in the table area;
determine that the detected entity matches an entity category of a plurality of entity categories, wherein the plurality of entity categories includes one or more of: an item of a medical laboratory examination, an object value for the item, a unit for the object value, a reference value for the item, and an abnormal indication; and
generate a label indicating the detected entity and the matched entity category.

11. The method of claim 8, wherein the question and answers module is configured to
determine a data attribute based on the question;
determine a location of one or more characters related to the data attribute in the text; and
extract data of the string data type or the quantity data type from the text based on the location.

12. The method of claim 1, further comprising outputting the questionnaire filled with the extracted data.

13. The method of any of claims 1-12, wherein the selected data extraction module is generated by training a machine learning model with a training data set of the data type, the training data set includes standard data obtained from a plurality of data records.

14. An electronic device, comprising:
at least one processing unit; and
a memory coupled to the at least one processing unit and storing computer program instructions therein, the instructions, when executed by the at least one processing unit, causing the electronic device to perform the method of any of claims 1-13.

15. A computer-readable storage medium having program code stored thereon, the program code configured, upon execution, to cause an apparatus to perform the method of any of claims 1-13.
